# EUROPÄISCHE PATENTANMELDUNG

(11) **EP 0 554 651 A1**
(43) Veröffentlichungstag der Anmeldung: **11.08.1993**
(21) Anmeldenummer: 92890268.3
(22) Anmeldetag: 21.12.1992
(51) Int. Cl.: C07C 67/56, C07C 69/54

(54) **Mittel und Verfahren zur Entfernung von Säureresten aus (Poly)esterifikationsprodukte enthaltenden Reaktionsgemischen**

(30) Priorität: 20.12.1991 AT 2543/91
(71) Anmelder: REICHHOLD CHEMIE GES. M.B.H., 1222 Wien (AT)
(72) Erfinder: Gombotz, Franz, A-1140 Wien (AT); Fucik, Ivan Thomas, Dr. Dipl.-Ing., A-1110 Wien (AT); Klimesch, Karl, Dipl.-Ing., A-2500 Baden b. Wien (AT)
(74) Vertreter: Pfeifer, Otto, Dipl.-Ing.

(57) **Zusammenfassung**

Die Erfindung betrifft ein Verfahren zur Entfernung von Säureestern aus (Poly)esterifikationsprodukte enthaltenden Reaktionsgemischen, worin ein weitgehend umgesetztes (Poly)esterifikationsgemisch mit einem Mittel auf Basis eines im Reaktionsgemisch nicht oder nur gering löslichen, partikelförmigen, basisch reagierenden Feststoffes innig in Kontakt gebracht wird und das genannte Mittel nach Absenken der Säurekonzentration des genannten Reaktionsgemisches gegebenenfalls vom Reaktionsgemisch abgetrennt wird, worin das In-Kontakt-Bringen mit dem genannten Mittel im Kreislauf mit einem unter 100°C siedenden Kreislaufmittel unter Inertatmosphäre und Inhibierung mit einem System auf Basis von Verbindungen des Phenoltyps, welche keine zur Salzbildung befähigten Hydroxylgruppen besitzen, in an sich bekannter Weise erfolgt.

## Beschreibung

Die Erfindung betrift ein Verfahren zur Entfernung von Säureresten aus (Poly)esterifikationsprodukte enthaltenden Reaktionsgemischen. Die Erfindung betrifft insbesondere solch ein Verfahren, worin ein weitgehend umgesetztes (Poly)esterifikationsgemisch mit einem Mittel auf Basis eines im Reaktionsgemisch nicht oder nur gering löslichen, partikelförmigen, basisch reagierenden Feststoffes innig in Kontakt gebracht wird.

In der vorliegenden Erfindung werden unter "(Poly)esterifikationsprodukte enthaltenden Reaktionsgemischen" (Poly)ester verstanden, welche durch Reste von Ausgangs-, Zwischenprodukten und gegebenenfalls durch Wasser und/oder Lösungsmittel verunreinigt sind.

Die Herstellung von (Poly)estern ist sehr oft durch die Schwierigkeit der Entfernung von nicht umgesetzten Säureresten gekennzeichnet. Es werden meist anspruchsvolle und zeitaufwendige Waschvorgänge verwendet bzw. langzeitige, gegebenenfalls mehrstufige Veresterungsprozesse eingesetzt. Sehr oft werden aufwendige Destillationen notwendig. Des öfteren müssen sogar Dünnschichtverdampfer verwendet werden, um die Säurereste weitgehendst zu entfernen.

Derartige Verfahren zur Entfernung der Säurereste sind jedoch meist sehr arbeitsintensiv, zeitraubend, mit hohen Kosten verbunden und häufig ökologisch nachteilig.

Beispielsweise wird in der Offenlegungsschrift DE 37 03 080 A1 die Herstellung von (Meth)-Acrylsäureester beschrieben, welche die Entfernung der überschüssigen (Meth)-Acrylsäure durch dreimalige Extraktion mit gesättigter Natriumhydrogencarbonat-Lösung und anschließendem Auswaschen mit Wasser, gefolgt von einer Trocknung über Natriumsulfat und Nachbehandlung mit Aktivkohle vorsieht. Diese Herstellung führt zwar zu weitgehend säurefreien (Meth)-Acrylsäureestern, hat jedoch den Nachteil eines aufwendigen Verfahrens (dreimalige Extraktion, Trocknung, Behandlung mit Aktivkohle ) mit ungünstigen ökologischen Aspekten (große Abwassermengen).

In der deutschen Auslegeschrift 1 241 375 wird der Acrylsäureester von überschüssiger Acrylsäure befreit, indem die Acrylsäure im Vakuum abdestilliert wird, der Rückstand in Pentan aufgennommen und dreimal mit Wasser gewaschen wird. Durch Waschen mit 10%iger Sodalösung und 5%iger NaOH werden die Reste von Säure und Katalysator entfernt. Nach dem Abtrennen der organischen Schicht wird das Pentan abdestilliert und der Rückstand nach Zugabe von Phenothiazin im Vakuum fraktioniert. Diese Methode vereinigt das Auswaschen der Säurereste mit der Entfernung durch Vakuumdestillation. Dies ist ein wirtschaftlich aufwendiges und ökologisch nachteiliges Verfahren. Durch dreimaliges Waschen mit Wasser wird dabei beispielsweise eine Menge an Waschwasser erhalten, die größer als die Menge des gewünschten Acrylsäureesters ist.

Daß nach der Vakuumdestillation noch ausgewaschen werden muß, zeigt ferner sehr deutlich, daß mit herkömmlicher Vakuumdestillation nur ein Teil der Säurereste entfernt werden kann und weitere Schritte folgen müssen. Dabei werden die (Poly)esterifikationsprodukte jedoch einer relativ großen thermischen Belastung ausgesetzt, was zu Qualitätseinbußen führen kann und weitere Kosten verursacht. Eine Nachinhibierung ist bei diesem Verfahren unumgänglich.

In der europäischen Patentschrift EP 0 054 105 B1 ist ein Verfahren zur Herstellung von (meth)-acrylsäuremodifizierten Polyestern beschrieben, worin die im Esterifikationsprodukt verbliebene freie Acrylsäure mit einer der Säuremenge äquivalenten Menge einer Mono- oder Diepoxidverbindung unter Verwendung von Triphenylphosphin als Katalysator bei 80°C bis 120°C bis zu einer Säurezahl von max. 10 mg KOH/g umgesetzt wird.Polyester können gemäß dieser Patentschrift zwar durch relativ einfache Verfahrensschritte von den entsprechenden Säureresten weitgehend befreit werden, aber eine Verunreinigung des Produktes durch Umsetzungsprodukte der Mono- oder Diepoxidverbindungen mit den zu entfernenden verbliebenen freien Säuren und Triphenylphosphin ist die Folge. Die Produkteigenschaften werden dadurch beeinflußt, die verbliebenen freien Säuren werden auf diese Weise nicht entfernt, sondern lediglich zu anderen hydroxylgruppenhältigen Produkten umgewandelt. Reine (Poly)ester können auf solche Weise nicht hergestellt werden.

In der US-PS 3 632 626 wird die Entfernung von phenolischen Polymerisationsinhibitoren, wie Hydrochinon, aus Alkenylmonomeren, wie Methylmetacrylat, mit alkalischen Ionentauschern beschrieben. Das beschriebene Verfahren ermöglicht es, auf Grund einer zweistufigen Behandlung mit unterschiedlichen Ionentauschern ein im wesentlichen Inhibitor-freies Monomerengemisch zu erhalten.

In der WO 91/08192 ist ein Verfahren zum Inhibitortausch in radikalisch reaktiven olefinisch ungesättigten Systemen beschrieben. Dabei wird von olefinisch ungesättigten Systemen ausgegangen, welche Inhibitoren enthalten, die Verbindungen des Phenoltyps mit zur Salzbildung befähigten Hydroxylgruppen sind.

Die DE-OS 1 517 901 hat ein Verfahren zum Aufbereiten von alkaliempfindlichen, beschränkt wasserlöslichen Flüssigkeiten mit Anionentauschern zum Gegenstand.

Die EP-A-0 376 089 hat ein sogenanntes Trockenneutralisierungsverfahren zum Gegenstand. Dabei wird zur Gewinnung von Reinprodukten, die auch ohne Destillation niedrige Rest-Säurezahlen mit niedrigen Farbzahlen verbinden sollen, eine Neutralisation mit feinpulvrigen Oxiden, Carbonaten und/oder Hydroxiden der Alkali- und/oder Erdalkalimetalle, gewünschtenfalls mit weiteren unlöslichen basischen Metalloxidverbindungen durchgeführt und nachfolgend die organische Flüssigphase von der feinpulvrigen Festphase abgetrennt.

Allen diesen Entgegehaltungen kann entnommen werden, daß ein sorgfältiges Auswählen der Verfahrensparameter für das Erreichen der jeweiligen Ziele erforderlich ist. Insbesondere in der EP-A-0 376 089 wird auf die Schwierigkeiten bei der Auswahl der einzelnen Parameter hingewiesen.

Ziel der vorliegenden Erfinfung ist daher ein Verfahren zur Entfernung von Säureresten aus (Poly) esterifikationsprodukte enthaltenden Reaktionsgemischen, das eine einfache und effiziente Herstellung von säurefreien oder säurearmen (Poly)estern ermöglicht und die oben genannten Nachteile der bekanten Verfahren überwindet.

Dieses Ziel wird erfindungsgemäß durch ein Verfahren erreicht, worin das In-Kontakt-Bringen eines Mittels auf Basis eines im Reaktionsgemisch nicht oder nur gering löslichen, partikelförmigen, basisch reagierenden Feststoffes mit dem weitgehend umgesetzten (Poly)esterifikationsgemisch im Kreislauf mit einem unter 100°C siedenden Kreislaufmittel unter Inertatmosphäre und Inhibierung mit einem System auf Basis von Verbindungen des Phenoltyps, welche keine zur Salzbildung befähigten Hydroxylgruppen besitzen, in an sich bekannter Weise erfolgt.

Durch das erfindungsgemäße Verfahren werden gleichzeitig geringe Farbzahlen und Säurezahlen unter 0,1 mg KOH erreicht. Ferner wird im Gegensatz zu Verfahren, die Stand der Technik sind, die Verwendung von Luft vermieden, welche in der Dampfphase als Inhibitor wirkt, jedoch im Hinblick auf die Explosionsgefahr ein enormes Sicherheitsrisiko darstellt. Weiters ist eine Anwendung zusätzlicher unlöslicher basischer Metalloxidverbindungen, welche keine Alkalimetall- oder Erdalkalimetallverbindungen sind, nicht erforderlich. Darüberhinaus können im erfindungsgemäßen Verfahren auch Ionentauscher an Stelle der genannten Alkalimetall- oder Erdalkalimetallverbindungen erfolgreich als Deacidierungsmittel verwendet werden.

Als unter 100°C siedendes Kreislaufmittel können Kohlenwasserstoffe oder Kohlenwasserstoffgemische, wie Cyclohexan oder Nappar 6, verwendet werden.

Die Inertatmosphäre wird vorzugsweise von Stickstoff oder Kohlendioxid gebildet und bei dem inhibierenden System handelt es sich bevorzugt um das System Phenothiazin/Nitrobenzol.

Der dacidierend wirkende Feststoff kann im erfindungsgemäßen Verfahren vorteilhafterweise in Partikelform von etwa 1 mm im Durchmesser verwendet werden und beispielsweise Calciumhydroxid, Calciumoxid, Magnesiumoxid, ein Träger mit einem darauf aufgebrachten basischen Medium, wie mit Calciumhydroxid beladene Kieselsäure (Kieselgur), oder ein makroporöser Ionenaustauscher sein. Trotz der Festform des Mittels und der niedrigen Konzentration der Säurereste der (Poly)esterifikationsprodukte erfolgt die Säureumsetzung überraschenderweise schnell und praktisch vollständig. Die Umsetzungsprodukte bleiben dabei meist an dem Mittel haften und können somit leicht entfernt werden.

Die praktische Durchführung des Verfahrens kann sowohl in einem Reaktor als auch in einer Art von Filtrationskolonne (ähnlich der Wasseraufbereitung) erfolgen.

Bei der Durchführung im Reaktor erfolgt die Entfernung des mit Umsetzungsprodukten kontaminierten (angereicherten) Mittels durch einfaches Abfiltrieren, während im Falle der nachgeschalteten Filtrationskolonne die Trennung direkt erfolgt.

Das Produkt, welches erfindungsgemäß behandelt wurde, ist praktisch säurefrei (SZ < 0,1 mg KOH/g), transparent und von anderen systemfremden Stoffen, wie beispielsweise Umsetzungsprodukten der verbliebenen freien Säuren mit Mono- bzw. Diepoxidverbindungen, sauerstoffaktiven Inhibitoren sowei Katalysatoren, frei.

Die erfindungsgemäße Lehre wird nachstehend an Hand von Beispielen näher erläutert, ohne jedoch darauf beschränkt zu sein. Es werden Herstellungen von ungesättigten Estern als Beispiel angeführt, bei welchen die Gefahr einer unerwünschten, vorzeitigen Polymerisation besteht.

### BEISPIEL I: Herstellung eines Trimethylolpropantriacrylates mit einem Säuregehalt von < 0,1 % mit Weißkalkhydrat als Deacidierungsmittel

In einem mit Rührer, Thermometer und Wasserabscheider versehenen Gefäß werden 425 g Trimethylolpropan, 700 g Acrylsäure und 482 g Nappar 6 vorgelegt. Bei Nappar 6 handelt es sich um Lösungsmittel aus naphthenischen Kohlenwasserstoffen. Als Polymerisationsinhibitoren werden 0, 7 g Phenothiazin und 0, 25 g Nitrobenzol zugegeben und N₂ wird übergeleitet.

Hierauf wird in Gegenwart von p-Toluolsulfonsäure als Veresterungskatalysator im Lösungsmittelkreislauf im Temperaturbereich von 75 - 90°C so lange umgesetzt, bis kein Reaktionswasser mehr anfällt und ein Umsatz von >85 %, vorzugsweise > 90 %, erreicht wird.

Die Säurezahl (SZ), bezogen auf Lösung des Reaktionsgemisches, beträgt 25 - 35 mg KOH/g.

Anschließend wird dem Reaktionsgemisch 83 g Weißkalkhydrat (Spezialkalk) bei 60 - 70°C zugegeben und das Gemisch wird 1 - 2 Stunden unter Lösungsmittelrückfluß bis zu einer SZ von 0 umgesetzt. Das gebildete Wasser kann dann gegebenenfalls im Lösungsmittelkreislauf entfernt werden, anderenfalls bleibt es im Weißkalkhydrat zurück.

Sobald eine vollständige Umsetzung erreicht ist, wird auf 30 - 40°C gekühlt, das Weißkalkhydrat wird abfiltriert und das Lösungsmittel wird im Vakuum bei max. 60°C vollständig entfernt. Das anfallende Lösungsmittel kann für eine neue Trimethylolpropantriacrylatherstellung verwendet werden.

Es wird ein Trimethylolpropantriacrylat erhalten,dessen Farbzahl < 1 µg Jod ist und dessen Restsäuregehalt < 0,1 % beträgt.

Die Säurezahl des TMPTA beträgt max. 0,1 mg KOH/g.

### BEISPIEL II: Herstellung von Trimethylolpropantrimethacrylat mit einem Säuregehalt von 0,1 % mit Calciumoxid als Deacidierungsmittel:

Wie im Beispiel I beschrieben, werden 402 g Trimethylolpropan, 790 g Methacrylsäure und 511 g Nappar 6 vorgelegt. Als Polymerisationsinhibitoren werden 0,7 g Phenothiazin und 0,25 g Nitrobenzol zugegeben. N₂ wird übergeleitet. Es wird - wie unter Beispiel I beschrieben - umgesetzt, mit Calciumoxid behandelt, filtriert und das Lösungsmittel im Vakuum entfernt. Es wird ein Trimethylolpropantrimethacrylat erhalten, dessen Farbzahl < 1 mg Jod ist und dessen Restsäuregehalt < 0,1 % beträgt.
Die Säurezahl des Trimethylolpropantrimethacrylates beträgt max. 0,1 mg KOH/g.

### BEISPIEL III: Herstellung eines Polyethylenglykol 400-diacrylates mit einem Säuregehalt von < 0,1 % mit Weißkalkhydrat als Deacidierungsmittel:

In einer wie im Beispiel I beschriebenen Apparatur werden 1.000 g Polyethylenglykol P-400, 367 g Acrylsäure und 586 g Nappar 6 vorgelegt. Als Polymerisationsinhibitoren werden 0,35 g Phenolthiazin und 0,13 g Nitrobenzol verwendet. N₂ wird übergeleitet. Es wird wie im Beispiel I beschrieben umgesetzt, mit Weißkalkhydrat behandelt, filtriert und das Lösungsmittel im Vakuum entfernt. Es wird ein Polyethylenglykol 400-diacrylat mit einem Restsäuregehalt von < 0,1 % und einer Farbzahl von < 1 mg Jod erhalten. Die Säurezahl des Polyethylenglykol 400-diacrylates beträgt max. 0, 2 mg KOH/g.

### BEISPIEL IV: Herstellung eines Tripropylenglykoldiacrylates mit einem Säuregehalt von < 0,1 % mit Magnesiumoxid als Deacidierungsmittel:

In einer wie im Beispiel I beschriebenen Apparatur werden 865 g Tripropylenglykol, 661 g Acrylsäure und 310 g Nappar 6 vorgelegt. Als Polymerisationsinhibitoren werden 0,4 g Phenothiazin und 0,2 g Nitrobenzol verwendet. N₂ wird übergeleitet.

Es wird wie im Beispiel I beschrieben umgesetzt, mit Weißkalkhydrat behandelt, filtriert und das Lösungsmittel im Vakuum entfernt. Es wird ein Tripropylenglykoldiacrylat mit einem Restsäuregehalt von < 0,1 % und einer Farbzahl von < 1 mg Jod erhalten.

Die Säurezahl des Tripropylenglykoldiacrylates beträgt max. 0,1 mg KOH/g.

### BEISPIEL V: Herstellung eines 1,5 Pentandioldiacrylates mit einem Restsäuregehalt von 0,1 %, mit Weißkalkhydrat als Deacidierungsmittel:

In einer Apparatur, wie im Beispiel I beschrieben, werden 520 g 1,5 Pentandiol, 735 g Acrylsäure und 537 g Nappar 6 vorgelegt. Als Polymerisationsinhibitoren werden 0,7 g Phenothiazin und 0,35 g Nitrobenzol zugegeben. N₂ wird übergeleitet.

Es wird wie im Beispiel I beschrieben umgesetzt, mit Weißkalkhydrat behandelt, filtriert und das Lösungsmittel im Vakuum entfernt. Es wird ein 1,5 Pentadioldiacrylat mit einem Restsäuregehalt von < 0, 1 % und einer Farbzahl von < 1 mg Jod erhalten.

Die Säurezahl des 1,5 Pentandioldiacrylates beträgt max. 0,1 mg KOH/g.

### BEISPIEL VI: Herstellung eines Triethoxytrimethylolpropantriacrylates mit einem Säuregehalt von < 0,1 % mit Weißkalkhydrat als Deacidie rungsmittel:

Wie im Beispiel I beschreiben werden 1.088 g triethoxyliertes Trimethylolpropan, 951 g Acrylsäure und 510 g Nappar 6 vorgelegt. Als Polymerisationsinhibitoren werden 1, 2 g Phenothiazin, und 0, 4 g Nitrobenzol zugegeben. N₂ wird übergeleitet.

Es wird wie im Beispiel I beschrieben umgesetzt, mit Weißkalkhydrat behandelt, filtriert und das Lösungsmittel im Vakuum entfernt. Es wird ein Triethoxytrimethylolpropantriacrylat mit einer Farbzahl von < 1 mg Jod erhalten, dessen Restsäuregehalt < 0,1 % beträgt. Die Säurezahl des Triethoxytrimethylolpropantriacrylates beträgt < 0,1 mg KOH/g.

### BEISPIEL VII: Herstellung eines Triethoxytrimethylolpropantriacrylates mit einem Säuregehalt < 0,1 %. Deacidierungsmittel: Kieselgur mit einer Deposition von Ca(OH)₂.

180 g Kieselgur werden mit 185 g Weißkalkhydratmilch (20%ig) vermengt und bei 105°C im Umluftschrank auf Gewichtskonstanz getrocknet. Das so hergestellte Deacidierungsmittel wird in eine Kolonne eingebracht, durch welche ein Esterifikationsprodukt ohne abgetrenntes Lösungsmittel mit einer Säurezahl von 25 bis 35 durchgeleitet wird.

Das nach der Kolonne erhaltene Produkt ist ein praktisch säurefreies Triethoxytrimethylolpropandioltriacrylat mit Lösungsmittel. Das Lösungsmittel wird dann in Vakuum bei maximal 60°C entfern und das entstandene Produkt weist eine Säurezahl von < 0,1 mg KOH/g und eine Farbzahl von < 1 mg Jod auf.

### BEISPIEL VIII: Herstellung eines Triethoxytrimethylolpropantriacrylates mit einem Säuregehalt von < 0,1 % durch Behandlung mit makroporösem Anionenaustauscher in OH-Form.

Ein Esterifikationsprodukt von Säurezahl 25 - 35, hergestellt wie in Beispiel VI, wird über eine Kolonne mit einem makroporösen, stark basischen Anionenaustauscher in OH-Form geführt und anschließend durch Vakuumdestillation bei Temperatur von max. 60°C vom Lösungsmittel befreit. Das erhaltene Produkt weist einen Säuregehalt von < 0,1 % und eine Farbzahl von < 1 mg Jod auf.

### BEISPIEL IX: Herstellung eines Trimethylolpropantriacrylates mit einem Säure gehalt von < 0,1 % durch Behandlung mit makroporösem, schwach saurem Kationenaustauscher in Na⁺-Form.

Ein Esterifikationsprodukt von Säurezahl 25 - 35, hergestellt wie in Beispiel I beschrieben, wird über eine Kolonne mit schwach sauren makroporösem Kationenaustauscher in Na⁺-Form geführt. Das anschließend erhaltene und durch Vakuumdestillation bei max. 60°C von Lösungsmittel befreite und filtrierte Produkt besitzt einen Säuregehalt von < 0,1 % und weist eine Farbzahl von < 1 mg Jod auf.

## Patentansprüche

1. Verfahren zur Entfernung von Säureestern aus (Poly)esterifikationsprodukte enthaltenden Reaktionsgemischen, worin ein weitgehend umgesetztes (Poly)esterifikationsgemisch mit einem Mittel auf Basis eines im Reaktionsgemisch nicht oder nur gering löslichen, partikelförmigen, basisch reagierenden Feststoffes innig in Kontakt gebracht wird und das genannte Mittel nach Absenken der Säurekonzentration des genannten Reaktionsgemisches gegebenenfalls vom Reaktionsgemisch abgetrennt wird, dadurch gekennzeichnet, daß das In-Kontakt-Bringen mit dem genannten Mittel im Kreislauf mit einem unter 100°C siedenden Kreislaufmittel unter Inertatmosphäre und Inhibierung mit einem System auf Basis von Verbindungen des Phenoltyps, welche keine zur Salzbildung befähigten Hydroxylgruppen besitzen, in an sich bekannter Weise erfolgt.

2. Verfahren nach Anspruch 1, dadurch gekennzeichnet, daß das unter 100°C siedende Kreislaufmittel Nappar oder Cyclohexan ist.

3. Verfahren nach Anspruch 1 oder 2, dadurch gekennzeichnet, daß die Inertatmosphäre von Stickstoff oder Kohlendioxid gebildet wird.

4. Verfahren nach einem der Ansprüche 1 bis 3, dadurch gekennzeichnet, daß das inhibierende System Phenothiazin und Nitrobenzol ist.
